(19) **Europäisches Patentamt / European Patent Office / Office européen des brevets**

(11) **EP 1 384 470 B2**

(12) **NEW EUROPEAN PATENT SPECIFICATION**
After opposition procedure

(45) Date of publication and mention
of the opposition decision:
**24.09.2014 Bulletin 2014/39**

(45) Mention of the grant of the patent:
**14.09.2011 Bulletin 2011/37**

(21) Application number: **03016504.7**

(22) Date of filing: **21.07.2003**

(51) Int Cl.:
*A61K 8/55* (2006.01)       *A61K 8/73* (2006.01)
*A61K 8/81* (2006.01)       *A61K 8/92* (2006.01)
*A61Q 19/10* (2006.01)

(54) **Skin cleansing composition**

Zubereitung zur Hautreinigung

Composition de nettoyage de la peau

(84) Designated Contracting States:
**DE FR GB**

(30) Priority: **22.07.2002 JP 2002212973**
**28.02.2003 JP 2003054842**
**25.04.2003 JP 2003121942**

(43) Date of publication of application:
**28.01.2004 Bulletin 2004/05**

(73) Proprietor: **KAO CORPORATION**
**Chuo-ku,**
**Tokyo (JP)**

(72) Inventor: **Yamazaki, Ritsuko,**
**Kao Cor.Res.Lab.**
**Tokyo 131-8501 (JP)**

(74) Representative: **HOFFMANN EITLE**
**Patent- und Rechtsanwälte**
**Arabellastrasse 4**
**81925 München (DE)**

(56) References cited:
**EP-A- 0 463 780       WO-A-98/04237**
**WO-A-03/039499       GB-A- 1 228 060**
**GB-A- 2 283 755       US-A- 5 154 849**
**US-A- 5 776 871       US-A- 5 910 472**
**US-B1- 6 172 019       US-B1- 6 348 188**

Remarks:
The file contains technical information submitted after
the application was filed and not included in this
specification

EP 1 384 470 B2

**Description**

Field of the Invention

The present invention relates to a skin cleansing composition, and, particularly, to a skin cleansing composition which has skin cleansing ability and also allows a powder and/or an oil in the composition to remain on the washed skin to impart a cosmetic effect by such a residue.

Background of the Invention

Skin cleansing is one of the basic procedures in cosmetic treatment of the skin. Generally, the skin is washed using a detergent such as a soap and then is conditioned using a lotion or a milky lotion, followed by applying make-up by using foundations or other color cosmetics, or skin care treatment by using a skin cream or a sunscreen cosmetic, so as to provide a desired color, glossiness and texture, or to nourish the skin or to prevent suntan, respectively.

Accordingly, in conventional procedures, a skin cleansing procedure is carried out separately from subsequent cosmetic procedures such as skin conditioning, applying make-up or other skin care treatment, and these procedures are conducted neither in parallel nor by one continuous action.

United States Patent 3,489,686 (hereinafter, USP '686) discloses a detergent composition containing (1) an organic surfactant, (2) a polyethyleneimine type water-soluble cationic polymer and (3) a powdery heavy metal salt of 2-pyridinethiol-1-oxide, and it also discloses that the surface deposition and hold of the heavy metal salt particles of 2-pyridinethiol-1-oxide which is an antibacterial and antidandruff agent are reinforced. This detergent composition is, however, used in shampoo applications with the intention of producing the aforementioned antibacterial and antidandruff effect but is not used to cleanse the skin such as a face or the like.

Further, United States Patent 5, 037, 818 (hereinafter, USP '818) discloses an aqueous washing composition containing (1) an anionic surfactant, (2) a non-cellulose type water-soluble cationic polymer and (3) water-insoluble particles (solid particles or emulsified oil particles of an anti-microbial agent). It also discloses that, on the contrary to the conventional detergents containing an anionic surfactant and a cationic polymer, wherein the anionic surfactant and the cationic polymer form a complex (Norda Briefs, No. 464, February, 1975), the deposition of the water-insoluble particles on the washed surface is promoted without precipitating the aforementioned complex in the invention composition.

However, this aqueous washing composition is also a detergent for shampoo use but not for washing the skin at a section where no hair is present. Also, the water-insoluble particles deposited on the section washed using this aqueous washing composition are used for imparting anti-microbial ability and a hair conditioning effect but not for imparting a cosmetic effect such as skin conditioning, changing the outward appearance of the skin and providing other skin care benefits.

Still further, Japanese Patent Application Laid-Open (JP-A) No . 55-38813 discloses a shampoo composition containing (1) at least one surfactant among anionic surfactants, amphoteric surfactants and alkylamine oxides, (2) a quaternary nitrogen-containing water-soluble polymer with a cationic charge density in a range of 0.0005 to 0.005 and (3) an oily additional substance, and also discloses that it imparts a natural gloss to the hair and exhibits an excellent conditioning ability.

Still further, Japanese Patent Application Laid-Open (JP-A) No. 7-53340 discloses a shampoo composition comprising (1) a surfactant among anionic surfactants, amphoteric surfactants and nonionic surfactants (2) a copolymer of diaryl dialkyl ammonium salt, (3) dimethylpolysiloxane or methylphenylpolysiloxane with a polymerization degree in the range of 3000 to 20000, (4) a hydrocarbon oil with 7 to 40 total carbon atoms or an ester oil with 7 to 40 total carbon atoms and (5) water, and also discloses that it has a good dispersibility of the high molecular silicone oil and is excellent in foaming ability and smoothness of the hair.

Still further, United States Patent 6,511,669 discloses that a cosmetic composition for cleansing which contains (1) a 2-hydroxylalkylcarboxyl type anionic surfactant or its salt, (2) a non-cellulose type cationic polymer with a cationic charge density of 2 meq/g or more may further contain (3) silicones, botanical oils, mineral oils and synthetic oils, and also discloses that it improves easiness of the combing, and smoothness and softness of the hair.

Still further, Japanese Patent Application Laid-Open (JP-A) No. 2001-131580 discloses a detergent composition containing (1) an anionic surfactant, (2) a cationic surfactant, (3) a silicone or a cationic polymer and (4) a glyceryl ether containing an alkyl or alkenyl group with 4 to 12 carbon atoms, and also discloses that it has excellent foaming performance and imparts a conditioning effect to the hair or body.

Still further, United States Patent 5,977,036 discloses a styling shampoo composition containing (1) a detergent surfactant selected from the group consisting of anionic surfactants, zwitter-ionic surfactants, amphoteric surfactants and mixtures thereof, (2) an organic cationic deposition polymer having a cationic charge density in the range of 0.2 meq/g to 7 meq/g and an average molecular weight in the range of 5,000 to 10,000,000, (3) a water-insoluble hair styling polymer, (4) a volatile water-insoluble carrier for the hair styling polymer, (5) a crystalline hydroxyl containing stabiling agent and (6) water, and also discloses that the composition provides improved spreading efficiency of the styling polymer onto hair, thus providing improved styling performance of the shampoo composition.

Still further, WO 98/04237 discloses a shampoo composition containing (1) a water-soluble surfactant selected from the group consisting of anionic, nonionic, amphoteric and cationic surfactants and mixtures thereof, (2) a cosmetic agent and/or a pharmaceutical active, (3) diamine-dipolyacid component and salts thereof and (4) water, and also discloses, as the cosmetic agent, silicone substances, cationic polymers, insoluble oils or the like. WO 98/04237 further discloses that the hair and/or the skin are improved in the its outward appearance and/or its feeling by the shampoo composition.

However, like USP '686 and USP '818, the references mentioned above merely disclose detergents for shampoo use. None of these references discloses a detergent for washing the skin at a section where no hair is present, nor discloses that the skin cleansing procedure and the cosmetic procedure such as skin conditioning, applying make-up or other skin care treatment are carried out in parallel or by one continuous action.

On the other hand, regarding a detergent composition with the intention of skin cleansing, for example, Japanese Patent Application Laid-Open (JP-A) No. 1-151510 discloses a detergent composition containing (1) a surfactant among sulfonic acid salts, sulfuric ester salts and N-acyl amino acid salts, (2) amphoteric surfactants of a particular betaine type, (3) low viscosity hydrophobic silicone oils and (4) polyoxyalkylene hydrogenated castor oils and/or its fatty acid esters, and discloses that it imparts a smooth feeling to the hair or the skin.

Further, WO 94/17166 discloses a personal cleansing composition containing (1) a surfactant among anionic surfactants, nonionic surfactants, amphoteric surfactants, zwitter-ionic surfactants, cationic surfactants and mixtures thereof, (2) a mixture of insoluble nonionic oils or waxes, (3) a fatty acid with a weight average chain length of 10 to 18 carbon atoms, (4) citric acid, its salt or a mixture thereof and (5) water, and also discloses that when the composition contains a hair or skin conditioning agent of a cationic or nonionic polymer, a conditioning effect is provided to the hair or skin.

However, these prior art compositions provide the skin with a moisturizing effect to the extent that they merely prevent skin roughening due to cleansing. Therefore, they are not enough to carry out skin cleansing and the skin conditioning in parallel or by one continuous action. In addition, these prior art compositions do not function as a make-up for changing the outward appearance of the skin.

## SUMMARY OF THE INVENTION

The present invention relates to a skin cleansing composition containing 5 to 40 % by weight of an anionic surfactant, 2 to 20 % by weight of a cationic polymer and 0.1 to 20 % by weight of a pigment powder, wherein the pigment powder is a flaky pigment powder.

## DETAILED DESCRIPTION OF THE INVENTION

All publications cited herein are hereby incorporated by reference.

Accordingly, the present invention relates to a skin cleansing composition which is capable of washing away soils, such as sebaceous soils on the skin, and also is capable of providing a cosmetic effect by allowing a powder, optionally supplemented by an oil, to efficiently remain on the washed skin. The cosmetic effect includes, for example, the make-up effect of changing or conditioning the outward appearance of the skin, such as the color, glossiness or texture of the skin and the skin-care benefits of imparting effects such as protection from ultraviolet rays, moisturizing or improvement in touch of the skin.

When the skin is washed using this skin cleansing composition, any one or more of a complex of the anionic surfactant and the cationic polymer, the anionic surfactant and the cationic polymer precipitate on the skin. Since the cosmetic powder, optionally supplemented by an oil, are associated with these precipitates, they efficiently remain as a residue on the skin to thereby impart a cosmetic effect to the skin.

A skin cleansing composition according to the present invention is a composition containing, as essential components, from 5 to 40% by weight of an anionic surfactant, from 2 to 20% by weight of a cationic polymer and from 0.1 to 20% by weight of a pigment powder, wherein the pigment powder is a flaky pigment powder.

The skin cleansing composition may be prepared in a liquid form such as a dispersion, suspension or emulsion such that the cosmetic powder, and mixtures of the cosmetic powder and a liquid oil and mixtures are dispersed in the aqueous phase dissolving the anionic surfactant and the cationic polymer.

In the present invention, the "skin-residual substance" is defined as a residue material on the skin, which can remain on the cleansed skin and provide a cosmetic effect such as a make-up effect or a skin care effect.

This skin cleansing composition has detergent properties. In addition, when the cleansing composition is applied to the skin for washing and rinsed using a large amount of water, any one or more of a complex of the anionic surfactant and the cationic polymer, the anionic surfactant or the cationic polymer precipitate on the skin. Since the cosmetic powder, optionally supplemented by an oil, is associated with these precipitates, theyefficientlyremain as a residue on the skin to thereby impart a cosmetic effect to the skin.

In embodiments of the present invention, various kinds of cosmetic effects can be imparted to a cleansed skin by using a skin-residual substance properly selected among the cosmetic powders, optionally supplemented the liquid oils, such as the ones described hereafter. It is to be noted that the "cosmeticizing" or "cosmetic effect" includes not only the make-up to change the outward appearance of the skin, but also various cosmetic treatments for the skin.

More specifically, there may be included: a case of using a make-up cosmetic such as a foundation or other color cosmetics to change color, gloss, texture or the like of the skin and finish in a desired appearance; or a case of conditioning the skin using a lotion, an emulsion or the like prior to applying the make-up; or a case of conducting the skin care treatment such as skin nourishing with a skin cream or the suntan prevention using a sunscreen cosmetics. Regarding the cosmetic effect by the powder, optionally supplemented by an oil, the effect of change in a skin appearance and the skin care benefits are particularly important as described hereafter.

The anionic surfactant is a major cleansing component of the skin cleansing composition according to the present invention. As anionic surfactants which may be used in embodiments of the present invention, alkyl sulfate, alkyl ether sulfate, fatty acid soap, monoalkyl phosphate, acyl L-glutamate and acyl isethionate are preferable. Among these compounds, a monoalkyl phosphate, acyl L-glutamate and fatty acid soap are preferable from the viewpoint of a tendency to form a complex having adhesiveness to the skin. Also, a monoalkyl phosphate and acyl L-glutamate are more preferable from the viewpoint of low irritation to the skin. The hydrophobic part (alkyl groups or acyl group) in each anionic surfactant preferably has 12 to 18 carbon atoms.

These compounds may be used in the form of, for example, a sodium salt, a potassium salt, ammonium salt or lower alkanolamine (e.g., mono, di and triethanolamine) salt.

Specific examples of the anionic surfactant may include sodium laurylsulfate, sodium myristylsulfate and sodium palmitylsulfate as the alkyl sulfates; sodium lauryl ether sulfate, sodium myristyl ether sulfate and sodium palmityl ether sulfate as the alkyl ether sulfates ; sodium salts, potassium salts, triethanolamine salts or arginine salts of lauric acid, myristic acid, palmitic acid or stearic acid as the fatty acid soaps; sodium salts, potassium salts, triethanolamine salts or arginine salts of monolaurylphosphoric acid as the monoalkyl phosphates ; sodium salts, potassium salts, triethanolamine salts or arginine salts of cocoylglutamic acid, lauroylglutamic acid or myristoylglutamic acid as the acyl L-glutamates ; and sodium salts, potassium salts, triethanolamine salts or arginine salts of cocoylisethionic acid or lauroylisethionic acid as the acyl isethionates.

The content of the anionic surfactant is designed to be generally 5 % by weight to 40 % by weight based on the total weight of the skin cleansing composition, and preferably 10 % by weight or more and more preferably 15 % by weight or more from the viewpoint of obtaining satisfactory cleansing effect, and preferably 35 % by weight or less and more preferably 30 % by weight or less and even more preferably 20% by weight or less from the viewpoint of weight balance with other components in consideration of stability when formulated and irritation to the skin.

The cationic polymer is a component necessary to impart residual property of the skin-residual substance after cleansing to the skin cleansing composition according to the present invention. If the anionic surfactant and the cationic polymer do not coexist in the composition, the cosmetic powder, optionally supplemented by an oil, to be the residue on the skin is washed away during rinsing so that the aimed cosmetic effect is not obtained. Specifically, the cationic polymer coexists without impairing the cleansing ability of the anionic surfactant to constitute the cleansing composition with a stable solution state. However, the cationic polymer is changed in its solution state by dilution during rinsing, with the result that it forms a complex with the anionic surfactant and precipitates, precipitates itself, or allows the anionic surfactant, which is not consumed in washing, to precipitate. Then, these precipitates associates with the skin-residual substance and therefore, the skin-residual substance adheres to the surface of the skin at the washed portion via the precipitates and remains on the surface of the skin to impart the cosmetic effect to the skin.

As the cationic polymer which may be used in embodiments of the present invention a homopolymer of dimethyldiallylammonium chloride or copolymers thereof with other co-monomers such as (meth)acrylic acid, (meth)acrylates and (meth) acrylamides, cationic cellulose and cationic guar gum are used from the view that they easily form a complex adhesive to the skin in combination with the anionic surfactant exemplified above and they are easily soluble in a formulated composition.

Here, it is to be noted that the word " (meth)acrylic" means "acrylic" and "methacrylic".

In particular, the homopolymer of dimethyldiallylammonium chloride or copolymers thereof with other co-monomers such as (meth)acrylic acid, (meth)acrylates and (meth)acrylamides are preferable from the view of allowing the powder and/or the oil to efficiently precipitate.

Specific examples may include: a homopolymer of dimethyldiallylammonium chloride (trademark: Merquat 100; manufactured by ONDEO Nalco), copolymer of dimethyldiallylammonium chloride and acrylamide (trademark: Merquat 550; manufactured by ONDEO Nalco), copolymer of dimethyldiallylammonium chloride and acrylic acid and acrylamide (trademark: Merquat 3330 and 3331; manufactured by ONDEO Nalco), cationic cellulose (trademark: Poise C-80M;

manufactured by Kao Corporation), and hydroxypropyltrimethylammonium guar gum (trademark: JR-125; manufactured by Union Carbide and Rabolgum CG-M; manufactured by Dainippon Pharmaceutical Co., Ltd.).

The cationic charge density of the cationic polymer used in embodiments of the present invention is preferably 0.001 or more from the viewpoint of easy formation of the complex with the anionic surfactant, and more preferably 0.003 or more from the viewpoint of easy precipitation of the complex when diluted, and more preferably 0.005 or more from the viewpoint of being excellent as an adhesive to the skin and an efficient remaining of the residual substance on the skin. Here, the cationic charge density (equivalent/g) means the number of cationic charges on the molecular weight of the monomer unit constituting the polymer.

The molecular weight of the cationic polymer used in embodiments of the present invention is preferably from 10,000 to 10,000,000 and more preferably from 100,000 to 1,000,000 from the viewpoint of remaining property of the residue material on the skin and a feel of the skin after cleansing. Here, the molecular weight means a weight average molecular weight measured by gel permeation chromatography (GPC), using tetrahydrofuran as a diluent; polystyrene as the standard; with a measuring instrument, for example, LC-2010 manufactured by Shimazu Corporation.

The content of the cationic polymer is designed to be usually from 0.5 % by weight to 20 % by weight based on the total weight of the skin cleansing composition, and preferably 1 % by weight or more and more preferably 2 % by weight or more from the view point of obtaining a satisfactory residual effect of the skin-residual substance, and preferably 15 % by weight or less and more preferably 10 % by weight or less from the viewpoint of weight balance with other components in consideration of good stability when formulated and a little irritation to the skin.

A blending ratio (surfactant/polymer) of the anionic surfactant to the cationic polymer is preferably in a range of from 1/1 to 50/1 from the viewpoint of allowing the skin-residual substance to efficiently remain, and more preferably in a range of from 3/1 to 30/1, and even more preferably in a range of from 5/1 to 20/1 from the viewpoint of cleansing ability and touch of the cleansing composition when used.

As the cosmetic powder, a pigment powder (make-up powder) is used. The pigment powder is a powder which adheres to the surface of the skin to change the outward appearance (e.g., color, glossiness and texture) having sufficient applicability to the skin.

The cosmetic powder is a flaky powders because the flaky powder is superior in the effect of providing a luster feel and smooth finish. Examples of flaky pigment powders include mica, talc, titanium mica and powders obtained by processing the aforementioned powders by surface treatment.

The surface of the cosmetic powder may be untreated or may be processed by hydrophobic treatment or hydrophilic treatment. However, the surface is preferably treated hydrophobically as aforementioned from the viewpoint of retaining a cosmetic effect over time.

The average particle diameter of the cosmetic powder may be measured by a laser diffraction/scattering method and is preferably from 0.1 $\mu$m to 100 $\mu$m and more preferably from 1 $\mu$m to 50 $\mu$m from the viewpoint of the scattering intensity of light, high coloring ability, masking ability for masking stains and the like and the stability of the skin cleansing composition.

The liquid oil, wich may supplement the cosmetic powder as a substance, is a component which adheres to and remains on the surface of the cleansed skin, and thus it prevents moisture from transpiring from the skin after washing to provide the moisturizing effect and also impart a good feeling of touch to the skin. Therefore, the liquid oil to be used in embodiments of the present invention is preferably a nonvolatile oil with a high moisturizing effect for preventing water vaporization. Further, the liquid oil is preferably a water-insoluble oil from the view that the prescribed oil is associated with a precipitate such as the complex of the anionic surfactant and the cationic polymer or the like to efficiently remain on the skin.

The liquid oil to be used in embodiments of the present invention preferably has a viscosity in a range of from 1 to 10,000 mPa · s to exhibit desired effects such as a remaining performance, a moisturizing effect and an improvement of feeling or touch. Here, the viscosity is a value to be measured at 25 °C using the BM type viscometer (e.x., manufactured by TOKIMEC INC.) with a BL adaptor rotating at 6 to 60 r.p.m.

The viscosity of the liquid oil is more preferably 10 mPa · s or more, and even more preferably 100 mPa · s or more to maintain the above mentioned moisturizing effect. Further, the viscosity is preferably preferably 5, 000 mPa · s or less, and is preferably 1, 000 mPa · s or less from the view of dispersibility and feeling of the cleansing composition when touch.

The liquid oil to be used in embodiments of the present invention is preferably a nonvolatile oil which has a viscosity of from 1 to 10,000 mPa · s and provides a high moisturizing effect. Here, the boiling point of the nonvolatile oil is preferably 80 °C or more under normal pressure. In particular, the liquid oil is preferably at least one kind selected from the group consisting of silicone oils, hydrocarbon oils, ester oils from the viewpoint of feeling to the skin when touched upon application and a high moisturizing effect. Among them, the hydrocarbon oil and the ester oil are further preferable in the viewpoint of efficiently providing the skin with the moisturizing effect.

Examples of silicone oils include dimethyl polysiloxane, octamethyl trisiloxane, decamethyl tetrasiloxane, methylphenyl polysiloxane, dimethyl polysiloxane having hydroxyl group, methylphenyl polysiloxane having hydroxyl group, polyether modified silicone and trimethyl siloxy silicic acid. Among them, dimethyl polysiloxane,methylphenyl polysiloxane, dimethyl polysiloxane having hydroxyl group and methylphenyl polysiloxane having hydroxyl group are preferable from the viewpoint of its feeling on the skin at the time of applying. Concrete examples of commercial products include the SH series available from Toray-Dow Corning Silicone Corporation and the KF series available from Shin-Etsu Silicone Corporation

Examples of hydrocarbon oils include light liquid isoparaffin type hydrocarbons and paraffin type hydrocarbons each of which has from 7 to 40 total carbon atom. Among them, a light liquid isoparaffin type hydrocarbon and a paraffin type hydrocarbon each from 10 to 30 total carbon atom are preferable from the viewpoint of its feeling at the time of applying to the skin. Concrete examples of commercial products include Isosol 400 available from Nihon Sekiyu Kagaku Corporation and the Pearleam EX available from NOF Corporation.

Examples of ester oils include those having from 7 to 40 carbon atoms, and more specifically isononyl isononanoate, isotridecyl isononanoate, isostearyl myristate, isostearyl palmitate, triglyceride isostearate, monoglyceride isostearate, neopenthylglycol dioctanoate, neopenthylglycol dicaprate, octyldodecyl lactiate, and diisostearyl malate. Among them, isononyl isononanoate, triglyceride isostearate, monoglyceride isostearate and neopenthylglycol dicaprate are preferable. Concrete examples of commercial products include Salacos 99 and Salacos 913 available from Nisshin Seiyu Co., Ltd., the Sunsoft Series available from Taiyo Chemical Corporation and Estemol N-01 available from Nisshin Seiyu Co., Ltd..

Examples of ether oils include monoalkyl glyceryl ethers such as monolauryl glyceryl ether, monomyristhyl glyceryl ether or monoisostearyl glyceryl ether; and dialkyl glyceryl ethers such as dilauryl glyceryl ether or diisostearyl glyceryl ether. Among them, monoisostearyl glyceryl ether and diisostearyl glyceryl ether are preferable from the viewpoint of its feeling at the time of using to the skin. Concrete examples of commercial products include GE-IS which is available from Kao Corporation.

As the skin-residual substance to be compounded in the cleansing composition of embodiments of the present invention, only one kind solely or two or more kinds of the cosmetic powder may be used, and the powder and the oil may be used in combination with each other.

The content of the skin-residual substance is designed to be usually from 0.1 % to 20 % by weight based on the total weight of the skin cleansing composition.

In the case of using cosmetic powder as a skin-residual substance, the content thereof is preferably 1 % by weight or more, and more preferably 2 % by weight or more based on the total weight of the skin cleansing composition, from the viewpoint of obtaining a sufficient concentration of the powder. The content of cosmetic powder is also preferably 15 % by weight or less, and more preferably 10 % by weight or less based on the total weight of the skin cleansing composition, from the viewpoint of weight balance with other components in consideration of stability when formulated and irritation to the skin.

On the other hand, in the case of using liquid oil as a further optional skin-residual substance, the content thereof is preferably 1 % by weight or more, and more preferably 5 % by weight or more based on the total weight of the skin cleansing composition, from the viewpoint of obtaining a sufficiently good feeling on the skin after applying. The content of the liquid oil is also preferably 20 % by weight or less, and more preferably 10 % by weight or less based on the total weight of the skin cleansing composition, from the viewpoint of the weight balance with other components in consideration of stability when formulated and irritation to the skin.

In the case of using cosmetic powder and liquid oil in combination as the skin-residual substance, each component can be used in the content as mentioned above.

The cleansing composition of embodiments of the present invention may further contain a component other than the above mentioned components, for example, a surfactant other than the anionic surfactant as a base material for the detergent; a polyhydric alcohol as a moisturizing agent such as glycerine, sorbitol, propylene glycol, butylene glycol or ethylene glycol; a water-soluble polymer other than the cationic polymer as a viscosity adjusting or thickening agent.

The cleansing composition of embodiments of the present invention also may contain another component in addition to the above components according to the need, and examples of the optional components include: solid oils and fats such as hydrocarbons, higher fatty acids, higher alcohols, esters or silicones; oil-soluble resins such as fluororesins, silicone resins, aromatic hydrocarbons, terpene resins, polybutene resins, isoprene resins, alkyd resins or polyvinylpyrrolidone based resins; organic ultraviolet absorber; make-up retentivity improver (e.g., fluorine-containing oil and zinc oxide); perfume;; anti-ance agent; antioxidant; anti-free radical agent; opacifier; stabilizer; emolient agent; hydroxy acid; anti-foaming agent; vitamins; ceramides; antiseptic; sequestering agent; inorganic salt; other active ingredients which are generally used for cosmetics; and organic solvent materials or volatile substances such as ethanol.

The skin cleansing composition according to the present invention may be prepared by weighing the anionic surfactant, the cationic polymer, the skin-residual substance selected among the above mentioned cosmetic powder, optionally supplemented by the above mentioned, liquid oil and, as required, other components and by mixing these components in water or an aqueous medium containing water as a major component and other water-soluble solvents such as an alcohol or the like, in a proper order in consideration of solubility and dispersibility of each component, followed by stirring. Here, the viscosity of the aqueous medium may be adjusted by adding a thickener.

The order of mixing and dissolving each component is not particularly limited insofar as a stable solution state of the anionic surfactant and the cationic polymer is obtained. Each amount of the anionic surfactant and the cationic polymer is desirably adjusted to the concentration and mixing ratio in which a cleansing effect or a residual effect for the skin-residual substance are satisfactorily obtained and both ob the above mentioned components can coexist while keeping a stable solution state.

In the skin cleansing composition according to the present invention obtained in this manner, the anionic surfactant and the cationic polymer coexist in a stable solution state due to a good balance thereof when the composition is stored after preparation.

The skin-residual substance is usually present in a dispersed state such as a suspension or emulsion form in the skin cleansing composition. Also it may be present in a precipitated state if it can be dispersed again by shaking. In the case where the skin-residual substance is a liquid oil, it may be solubilized in the skin cleansing composition.

When the skin cleansing composition is used, an appropriate amount of the composition is applied to the skin at a section of the skin where a cosmetic effect is desired and the applied section is massaged (specifically, rubbed with lightly pressing or with massaging). Thus, soils on the skin are removed.

Then, the massaging is continued while adding an appropriate amount of water to the same section, such that the skin cleansing composition is not totally washed away. The skin cleansing composition is diluted with water, thereby destroying the balance of the system in which the anionic surfactant and the cationic polymer were kept in a stable solution state, and one or more of a complex of the anionic surfactant and the cationic polymer, the anionic surfactant and the cationic polymer precipitate, with the result that the skin-residual substance adheres to the washed section of the surface of the skin by these precipitates. Then, when the washed section is rinsed by adding a large amount of water, soils on the skin are removed but the skin-residual substance remains, whereby a cosmetic effect can be imparted to the skin.

Moreover, the retention of the cosmetic effect is also excellent, because the skin-residual substance left as a residue after washing is neither removed nor peeled-off from the surface of the skin even after being rinsed and dried.

The cosmetic effect to be provided by the skin cleansing composition of embodiments of the present invention varies according to the kind or a combination of the cosmetic powder and the liquid oil used as the skin-residual substance. For example, in the case of formulating a pigment powder changing the outward appearance of the skin, a powder having sunscreening ability and an oil having a moisturizing effect in combination, the skin after washing can be provided with all effects of a make-up effect by the change in appearance of the skin, a sunscreen effect from ultraviolet rays and an effect of improvement in feeling of the skin at the same time.

In the skin cleansing composition according to the present invention, the skin-residual substances may be used either solely or in combination in an optional amount irrespective of types, and therefore the composition can impart various cosmetic effects to the washed skin and control desired effect.

In the case of using a pigment powder as the cosmetic powder, the outward appearance of the skin after washing can be changed in such manner that, for example, a hue is changed so as to intensify whiteness, blackness or redness, or an uniform or lamella-wise glossiness is provided, or pores, spots or freckles, fine wrinkles or rugulae are covered and corrected. Also, the skin cleansing composition according to embodiments of the invention may be intended not to target at a significant make-up effect, but to change the outward appearance of the skin to the extent that washed skin is controlled. The skin cleansing composition is not deteriorated in cleansing ability although it provides various effects as described above.

It maybe difficult to evaluate the cosmetic effect obtained by the skin cleansing composition of embodiments of the present invention quantitatively by using a specific parameter. It is however possible to make quantitative evaluations by measuring the color difference ($\Delta E$) between the skins before and after washing.

When a skin cleansing composition according to the present invention has the purpose of changing the hue of the skin primarily in such a case of containing a pigment powder as the skin-residual substance, the skin cleansing composition is applied to a circular section of 25 mm in diameter ($\phi$) on the surface of the inner side of the human forearm in an amount containing 1 mg of the skin-residual substance, and the circular section is massaged 10 strokes using fingers and rinsed with water while massaging 10 strokes. Then each color value is measured before applying the skin cleansing composition and after washing, thereby finding a color difference ($\Delta E$) of the applied surface (hereinafter, it is referred as "direct method".).

The obtained color difference is preferably 2 or more and more preferably 3 or more from the viewpoint of changing the outward appearance of the skin.

Here, the color difference ($\Delta E$) means thedifferencebetween two color values $L_1 a_1 b_1$ (before washing) and $L_2 a_2 b_2$ (after washing) which are measured using a color indication by the CIE 1976 (L*a*b*) color system and calculated according to the following equation.

< Equation of calculation of color difference (ΔE)>

$$\Delta E = \{(L_1 - L_2)^2 + (a_1 - a_2)^2 + (b_1 - b_2)^2\}^{1/2}$$

The color value by the L*a*b* color indication system can be measured using a commercially available color meter (e.g., CR-200 manufactured by Minolta Co., Ltd.).

On the other hand, when the skin cleansing composition according to the present invention has the purpose of mainly changing visual elements, such as glossiness or uniformity of the skin, other than the hue, by using a pearlescent pigment as the skin-residual substance, or when the purpose of providing skin care benefits accompanied by no change in the outward appearance of the skin by using a sunscreen powder or an oil as the skin-residual substance, the cosmetic effect may be quantitatively evaluated indirectly by a color difference (ΔE) obtained by adding a fixed amount of titanium oxide or iron oxide red as a marker to the skin cleansing composition.

In this case, titanium oxide or iron oxide red is added as a marker in a ratio of 5 % by weight to the skin cleansing composition and dispersed to prepare a marker-containing sample for evaluation. Thereafter, the sample for evaluation is applied to a circular section of 25 mm in diameter (φ) on the surface of the inner side of the human fore-arm in amount containing 1 mg of the maker and the circular section is massaged 10 strokes using fingers and rinsed by with water while massaging 10 strokes. The each color value is measured before applying the marker-containing sample for evaluation and after washing, thereby determining a color difference (ΔE) of the applied surface (hereinafter, it is referred as "marker method".).

The reason why two types of markers, namely, titanium oxide and iron oxide red are selectively used is that the coloring effect produced by the skin cleansing compositions as subjects to evaluation is diverse and, therefore, there is therefore the case where the color of the marker is offset, which makes it difficult to specify only one marker. Also, the reason why titanium oxide and iron oxide red are specified as markers as mentioned above is that if these two types are used, the coloring effect of at least one type is not offset and both are easily available and it is therefore considered that the both can be adequate indexes for evaluating the residual effect.

In the case of evaluating the powder residual effect by the marker method, it is preferable that a color difference (ΔE) between color values before and after washing either by using titanium oxide or iron oxide red is 5 or more and more preferably 10 or more from the viewpoint of obtaining a satisfactory make-up effect.

As mentioned above, the skin cleansing composition according to embodiments of the present invention is a new type of skin cleansing composition which functions as a detergent for the skin and functions to provide a cosmetic effect. When the skin is washed using the skin cleansing composition, the composition can wash away soils, such as sebaceous soils on the skin, and also allows the skin-residual substance with the intention of providing a cosmetic effect to remain on the washed skin and thus provides such cosmetic effect due to the skin-residual substance.

The cosmetic effect to be provided after washing varies according to the skin-residual substance. For example, when the skin-residual substance is a pigment powder, an effect to change or control the outward appearance of the skin such as color, glossiness and texture or the like is provided. Further, when the skin-residual substance is a sunscreen powder, an effect to prevent suntan is provided, and when the skin-residual substance is a moisturizing oil, an effect to provide skin care benefits such as improvement in feeling of the skin is provided.

Also, the residue on the skin is not easily removed nor peeled-off and therefore has retentivity of the cosmetic effect.

Also, because the skin cleansing composition according to embodiments of the present invention provides a cosmetic effect described above such as to make the outward appearance of the skin beautiful, to protect the skin from ultraviolet rays and to provide a moisturizing effect as well as cleansing effect of the skin, an advantage of shortening the time for cosmetic treatment to the skin is also obtained.

Also, the skin cleansing composition according to embodiments of the present invention allows desired skin-residual substances to be formulated in a desired combination and a desired amount. Therefore, particularly when using a pigment powder, the outward appearance of the skin after washing can be changed in various states and also finely controlled.

Further, when a moisturizing oil is used as a further optional skin-residual substance, a good feeling of the skin such as a moisturized feeling can be retained, and therefore application of a basic cosmetics such as a lotion is not necessary after washing the skin.

Thus the skin cleansing composition according to embodiments of the present invention can attain skin cosmetic effect by washing the face. That is, it is preferably used as a face cleansing composition which allows a substance providing benefits such as adding color by the application of a foundation, protection from ultraviolet rays, whitening, moisturizing or the like to remain on the skin after washing.

EXAMPLE

The following examples further describe and demonstrate embodiments of the present invention. The examples are given solely for the purpose of illustration and are not to be construed as limitations of the present invention.

Example A series

(Examples A1 to A14)

An anionic surfactant and a cationic polymer were weighed according to the ratios shown in Table A1 and added to water, followed by enough stirring at 50°C to prepare an uniform transparent solution. A predetermined cosmetic powder was further added to the transparent solution and dispersed with stirring to obtain a skin cleansing composition. Using this skin cleansing composition, the residual property of the powder was evaluated.
Also, in each example, the surfactant-polymer solution before adding the cosmetic powder was evaluated for precipitation when diluted and detergency.

(Comparative Examples A1 to A9)

An anionic or cationic surfactant, a cationic or anionic polymer and a cosmetic powder were weighed according to the ratios shown in Table A2. These compositions and surfactant-polymer solutions containing no cosmetic powder were evaluated in the same procedures as in Example A1.
The origin of the used materials are shown below.

    * 1 Monoalkyl phosphate: trademark: MAP 20H, manufactured by Kao Corporation.
    * 2 Potassium laurate: manufactured by Kanto Kagaku.
    * 3 Sodium lauryl ether sulfate: trademark: Emal 227C, manufactured by Kao Corporation.

    * 4 Potassium N-coco acyl L-glutamate: trademark: Amisoft CK11, manufactured by Ajinomoto Co., Ltd.
    * 5 Homopolymer of dimethyldiallylammonium chloride : trademark: Merquat 100 (weight average molecular weight: about 150,000, cationic charge density: 0.0066), manufactured by ONDEO Nalco.
    * 6 Cationic cellulose: trademark: Poise C-80M (weight average molecular weight : about 800, 000, cationic charge density : 0.0026), manufactured by Kao Corporation.
    * 7 Cationic guar gum: trademark: JR-125 (weight average molecular weight: about 250,000, cationic charge density: 0.0028), manufactured by Union Carbide.
    * 8 Titanium oxide: trademark: Tipaque CR50 (average particle diameter: 0.2 to 0.3 $\mu$m), manufactured by Ishihara Sangyo Co., Ltd.
    * 9 Mica: trademark: Mica Y-2300 (average particle diameter: 18 $\mu$m), manufactured by Yamaguchi Mica Co., Ltd.
    * 10 Talc: trademark: Talc MMR (average particle diameter: 4.4 $\mu$m or less), manufactured by Asada Seifun Co., Ltd.
    * 11 Polymethylmethacrylate (PMMA) particle: trademark: Microsphere MP-2200 (average particle diameter: 0.4 $\mu$m), manufactured by Matsumoto Yushi-Seiyaku Co., Ltd.
    *12 Lauryltrimethylammonium chloride: trademark: Quatamine 24P, manufactured by Kao Corporation.
    * 13 Polyethylene oxide: trademark: Alkox E-100, manufactured by Meiwa Kagaku.
    * 14 Sodium arginate (500 to 600 cp) : manufactured by Wako Pure Chemical Industries, Co., Ltd.

Table A1

| | Example No. | 1+ | 2 | 3 | 4+ | 5 | 6 | 7+ | 8 | 9 | 10 | 11 | 12 | 13 | 14 | 15+ | 16+ | 17+ |
|---|---|---|---|---|---|---|---|---|---|---|---|---|---|---|---|---|---|---|
| | Composition (wt%) | | | | | | | | | | | | | | | | | |
| Anionic Surfactant | MAP *1 | 20 | 20 | 20 | 20 | 20 | 20 | | | | | | | | | 20 | 20 | 2 |
| | Potassium laurate *2 | | | | | | | 15 | 15 | 15 | 15 | | | | | | | |
| | Sodium lauryl ether sulfate *3 | | | | | | | | | | | 15 | 20 | 20 | | | | |
| | Potassium N-coco acyl L-glutamate *4 | | | | | | | | | | | | | | 20 | | | |
| Cationic Polymer | Homopolymer of | | | | | | | | | | | | | | | | | |
| | dimethyldiallylammonium chloride *5 | 5 | 5 | 5 | 5 | | | 5 | 5 | | | 4 | | | 5 | 0.1 | 5 | 5 |
| | Cationic cellulose *6 | | | | | 5 | | | | 6 | | | 5 | | | | | |
| | Cationic guar gum *7 | | | | | | 5 | | | | 5 | | | 5 | | | | |
| Makeup Powder | Titanium oxide *8 | 0.1 | | | 2 | | | 2 | | | | | | | 2 | 0.05 | 2 | |
| | Mica *9 | | 10 | | | 10 | 10 | | 10 | 10 | 10 | 10 | 10 | 10 | 10 | | | |
| | Talc *10 | | | 10 | | | | | | | | | | | | | | |
| | PMMA particle *11 | | | | 4 | | | | | | | | | | | | | |
| | Purified Water | B a l a n c e | | | | | | | | | | | | | | | | |
| Evaluation | | | | | | | | | | | | | | | | | | |
| Precipitation characteristics when diluting | | P | P | P | P | P | P | P | P | P | P | P | P | P | P | N | P | N |
| Skin cleansing ability | | ○ | ○ | ○ | ○ | ○ | ○ | ○ | ○ | ○ | ○ | ○ | ○ | ○ | ○ | ○ | ○ | Δ |
| Residual property of a powder (Direct method) | | ○ | ○ | ○ | ○ | ○ | ○ | ○ | ○ | ○ | ○ | ○ | ○ | ○ | ○ | Δ | Δ | Δ |
| Residual property of a powder (Marker method) | | ○ | ○ | ○ | ○ | ○ | ○ | ○ | ○ | ○ | ○ | ○ | ○ | ○ | ○ | Δ | Δ | Δ |

** P:Present, N:None
+ Reference Example

EP 1 384 470 B2

Table A2

| Comparative Example No. | | 1 | 2 | 3 | 4 | 5 | 6 |
|---|---|---|---|---|---|---|---|
| Composition (wt%) | | | | | | | |
| Anionic Surfactant | MAP *1 | 20 | | 20 | 20 | 20 | |
| | Potassium laurate *2 | | | | | | |
| | Sodium lauryl ether sulfate *3 | | | | | | |
| Cationic Surfactant | Lauryl trimethyl ammonium chloride *12 | | | | | | 20 |
| Cationic Polymer | Homopolymer of dimethyldiallylammonium chloride *5 | | 5 | 5 | | | 5 |
| | Cationic cellulose *6 | | | | | | |
| | Cationic guar gum *7 | | | | | | |
| Nonionic Polymer | Polyethylene oxide *13 | | | | 5 | | |
| Anionic Polymer | Sodium Arginate *14 | | | | | 5 | |
| Makeup Powder | Titanium oxide *8 | 2 | 2 | | 2 | 2 | 2 |
| | Mica *9 | | | | | | |
| | Talc *10 | | | | | | |
| | PMMA particle *11 | | | | | | |
| Purified Water | | Balance | | | | | |
| Evaluation | | | | | | | |
| Precipitation characteristics when diluting ** | | N | N | P | N | N | N |
| Skin cleansing ability | | ○ | × | ○ | ○ | ○ | ○ |
| Residual property of a powder (Direct method) | | × | × | × | × | × | × |
| Residual property of a powder (Marker method) | | × | × | ○ | × | × | × |
| ** P:Present, N:None | | | | | | | |

(Evaluation)

With regard to the compositions obtained in each example and comparative example, precipitation characteristics when the composition was diluted, skin cleansing ability and the residual property of the powder were evaluated by the following procedures. The results of the evaluation are shown in Tables A1 and A2.

(1) Precipitation characteristics when diluting

2 g of the surfactant-polymer solution obtained in each example and comparative example was diluted with 8g of ion exchange water and the diluted solution was stirred for 5 minutes. This sample was allowed to stand for 5 minutes and then visually observed to judge whether precipitates were present or not. The criterion for the judgment is as follows.

<Criterion for evaluation of precipitation characteristics>

* None: The solution was a colorless, transparent and single-phase solution.
* Present: The solution was changed to a state other than a colorless, transparent and single-phase solution (for example, cloudy, separated into two-phases or precipitates were produced) .

(2) Skin cleansing ability

A model sebum having the following composition in which carbon black (Mitsubishi Chemical Co., Ltd.) was dispersed was prepared. 20 mg of the model sebum was applied to the inner side of the human fore-arm and spread to a circle of 25 mm in diameter ($\phi$). 30 mg of the surfactant-polymer solution obtained in each example and comparative example was dripped on this applied section, washing was carried out while massaging for 20 strokes, then the applied section was rinsed with city water and air-dried

Table A3

| | | | |
|---|---|---|---|
| Model Sebum | Squalane *15 | 9.0% | 100 parts by weight |
| | Myristyl myristate *16 | 24.0% | |
| | Cotton seed oil *17 | 47.0% | |
| | Cholesterol *18 | 2.0% | |
| | Cholesteryl palmitate *19 | 2.0% | |
| | Lauric acid *20 | 0.2% | |
| | Myristic acid *21 | 2.5% | |
| | Palmitic acid *22 | 6.0% | |
| | Stearic acid *23 | 0.9% | |
| | Oleic acid *24 | 6.4% | |
| Washing Marker | Carbon black *25 | | 5 parts by weight |

The origin of the used materials are shown below.

* 15 Squalane: manufactured by Wako Pure Chemical Industries, Ltd.
*16 Myristyl myristate: trademark: Exeparl MY-M, manufactured by Kao Corporation.
*17 Cotton seed oil: manufactured by Kanto Chemical Co., Ltd.
*18 Cholesterol: manufactured by Wako Pure Chemical Industries, Ltd.
*19 Cholesteryl palmitate : manufactured by Tokyo Kasei Kogyo Co., Ltd.
*20 Lauric acid: trademark: Lunac L-98, manufactured by Kao Corporation.
*21 Myristic acid: trademark: Lunac MY-98, manufactured by Kao Corporation.
*22 Palmitic acid: trademark: Lunac P-95, manufactured by Kao Corporation.
*23 Stearic acid: trademark: Lunac S-98, manufactured by Kao Corporation.
*24 Oleic acid: manufactured by Wako Pure Chemical Industries, Ltd.
*25 Carbon black: manufactured by Mitsubishi Chemical Co., Ltd.

The chromaticity of the arm at each point before the above washing procedures (before the model sebum was applied), before washing (after the model sebum was applied) and after washing (after rinsing and air-drying) was measured using a chromatic color difference meter CR-200 (MINOLTA) in terms of the color indication of the Lab system. Then, the chromaticity before treatment was used as a standard value ($L_{st}a_{st}b_{st}$) and the color difference ($\Delta E$) between each chromaticity (Lab) obtained before and after washing and the standard value was calculated from the following equation.

<Equation for calculating a color difference $\Delta E$>

$$\Delta E = \{(L - L_{st})^2 + (a - a_{st})^2 + (b - b_{st})^2\}^{1/2}$$

Then, from the color difference between the chromaticity before treatment and the chromaticity before washing and the color difference between the chromaticity before treatment and the chromaticity after washing, the washing rate of the model sebum was calculated from the following equation to evaluate according to the following criterion.

<Equation for calculating a washing rate>

```
    Washing rate (%) = (1 - (color difference after
washing/color difference before washing)) x 100
```

<Criterion for Evaluation of cleansing ability>

Washing rate < 70%: x
70% ≤ Washing rate < 80%: Δ
80% ≤ Washing rate < 90%: ○

900 ≤ Washing rate: ◎

(3) Residual property of the powder (Direct method)

30 mg of the skin cleansing composition obtained in each example and comparative example was dripped on the inner side of the human fore-arm and spread to a circle of 25 mm in diameter (φ). Washing was carried out while massaging for 10 strokes and an additional 10 strokes by adding water, and then the applied section was rinsed with city water. Color differences obtained before and after washing were found in the same method as in the aforementioned evaluation of the cleansing ability to evaluate in contrast with the following criterion.

<Criterion for Evaluation of the residual property of a powder>

ΔE < 1: ×
1 ≤ ΔE < 2: Δ
2 ≤ ΔE < 3: ○

3 ≤ ◎ ΔE:

(4) Residual property of the powder (marker method)

5 parts by weight of titanium oxide (Tipaque CR50; manufactured by Ishihara Sangyo Co., Ltd.) was added to and dispersed in 100 parts by weight of the skin cleansing composition obtained in each example and comparative example to obtain a dispersed mixture, which was used as a sample for evaluation. 30 mg of the sample for evaluation was dripped on the inner side of the human fore-arm and spread to a circle of 25 mm in diameter (φ). Washing was carried out while massaging for 10 strokes and an additional 10 strokes by adding water, and then the applied section was rinsed with city water. Color differences obtained before and after washing were found in the same method as in the aforementioned evaluation of cleansing ability to evaluate in contrast with the following criterion.

<Criterion for Evaluation of the residual property of a powder (marker method)>

ΔE < 2: x
2 ≤ ΔE < 5: Δ
5 ≤ ΔE < 10: ○

10 ≤ ΔE: ◎

Example B series

(Reference Examples B1 to B16)

According to the ratios shown in Table B1, a skin cleansing composition containing an oil as the skin-residual substance was obtained. Then, using the obtained skin cleansing composition, the residual property of the oil and feel of the skin after washing was evaluated. Procedures for preparing the skin cleansing composition was the same as in the above described Example A1 except that the cosmetic powder of the Example A1 was changed to an oil.
Also, in each example, the surfactant-polymer solution before adding the oil was evaluated for precipitation when diluted and detergency.

(Comparative Examples B1 to B6)

An anionic surfactant, a cationic or nonionic or anionic polymer and an oil were weighed according to the ratios shown in Table B2. Skin cleansing compositions and surfactant-polymer solutions containing no oil were evaluated in the same procedures as in Example B1.

(Comparative Examples B7 to B10)

Each component was weighed according to the ratios shown in Table B3, and each of Skin cleansing compositions was evaluated in the same procedures as in Example B1.
The origin of the used materials are shown below.

* 26 Monoalkyl phosphate: trademark: MAP 20H, manufactured by Kao Corporation.
* 27 Potassium laurate: manufactured by Kanto Kagaku.
* 28 Potassium N-coco acyl L-glutamate: trademark: Amisoft CK11, manufactured by Ajinomoto Co., Ltd.
* 29 Sodium lauryl ether sulfate: trademark: Emal 227C, manufactured by Kao Corporation.
* 30 Homopolymer of dimethyldiallylammonium chloride: trademark: Merquat 100 (weight average molecular weight: about 150,000, cationic charge density: 0.0066), manufactured by ONDEO Nalco.
* 31 Cationic cellulose: trademark: Poise C-80M (weight average molecular weight: about 800,000, cationic charge density: 0.0026), manufactured by Kao Corporation.
* 32 Cationic guar gum: trademark: JR-125 (weight average molecular weight: about 250,000, cationic charge density: 0.0028), manufactured by Union Carbide.
* 33 polydimethyl siloxane: trademark: silicone TSF-451 (viscosity: 3,000 mPa · s), manufactured by GE Toshiba Silicone Co., Ltd.
* 34 neopenthylglycol dicaprate: trademark: Estemol N-01 (viscosity: 19 mPa · s), manufactured by Nisshin Seiyu Co., Ltd.
* 35 squalane: (viscosity: 15 mPa · s), manufactured by Wako Pure Chemical Industries, Co., Ltd.
* 36 Polyethylene oxide: trademark: Alkox E-100, manufactured by Meiwa Kagaku.
* 37 Sodium arginate (500 to 600 cp): manufactured by Wako Pure Chemical Industries, Co., Ltd.
* 38 carnauba wax: trademark: Purified Carnauba Wax,(viscosity
> 1,000,000 mPa · s; out of the measuring limit), manufactured by Cerarica NODA Co., Ltd.
* 39 Sodium $\alpha$ - olefin sulfonate: trademark: Elfun OS46, manufactured by Goldwell Corporation.
* 40 betaine type amphoteric surfactant: trademark: Amphitol 20AB, manufactured by Kao Corporation.
* 41 N-Lauryl-$\beta$-aminopropionate: trademark: Delifat, Henckel Corporation.
* 42 copolymer of dimethyldiallylammonium chloride: trademark: Merquat 550 (weight average molecular weight: about 150,000, cationic charge density: 0.0033), manufactured by ONDEO Nalco.
* 43 maleic soybean oil: trademark: Cerafil GA, manufactured by Van Dyke Corporation.
* 44 myristic acid: trademark: Lunac MY-98, manufactured by Kao Corporation.
* 45 sodium citrate dihydrate: manufactured by Iwata Chemical Corporation.

Table B1

| Referecence Example No. | | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|---|
| Composi t i on (wt%) | | | | | | | | | | | | | | | | | | |
| Anionic Surfactant | MAP *26 | 20 | 20 | 20 | 20 | 20 | | | | | | | | | 20 | 20 | 2 |
| | Potassium laurate *27 | | | | | | 15 | 15 | 15 | | | | | | | | |
| | Potassium N-coco acyl L-glutamate *28 | | | | | | | | | 30 | 30 | | | | | | |
| | Sodium lauryl ether sulfate *29 | | | | | | | | | | | 15 | 20 | 20 | | | |
| Polymer | Homopolymer of dimethyldiallylammonium chloride (Cationic charge density: 0.0066) *30 | 5 | 5 | 5 | | | 5 | | | 4 | | 4 | | | 0.1 | 5 | 5 |
| | Cationic cellulose (Cationic charge density: 0.0026) *31 | | | | 5 | | | 5 | | | 4 | | 5 | | | | |
| | Cationic guar gum (Cationic charge density: 0.0028) *32 | | | | | 5 | | | 5 | | | | | 5 | | | |
| Water-insoluble oil | Polydimethyl siloxane (3000mPa.s) *33 | 5 | | | 5 | | | | | | | | | | 2 | 0.1 | 2 |
| | Neopenthylglycol dicaprate *34 | | 5 | | | 5 | 5 | 5 | 2 | 4 | 10 | 1 | 5 | 5 | | | |
| | Squalane *35 | | | 5 | | | | | | | | | | | | | |
| | Purified Water | B a l a n c e | | | | | | | | | | | | | | | |
| Evaluation | | | | | | | | | | | | | | | | | |
| Precipitation characteristics when diluting ** | | P | P | P | P | P | P | P | P | P | P | P | P | P | P | P | P |
| Skin cleansing ability | | ○ | ○ | ○ | ○ | ○ | ○ | ○ | ○ | ○ | ○ | ○ | ○ | ○ | ○ | ○ | ○ |
| Residual property of the oil | | ○ | ○ | ○ | Δ | Δ | ○ | Δ | Δ | ○ | ○ | | ○ | Δ | Δ | ○ | Δ |
| Feel of the skin after washing | | ○ | ○ | ○ | ○ | ○ | ○ | ○ | ○ | ○ | ○ | ○ | ○ | ○ | Δ | Δ | Δ |
| Moisturizing effect | | ○ | ○ | ○ | ○ | ○ | ○ | ○ | ○ | ○ | ○ | ○ | ○ | ○ | ○ | ○ | ○ |
| ** P:Present. N:None | | | | | | | | | | | | | | | | | |

Table B2

| | Comparative Example No: | 1 | 2 | 3 | 4 | 5 | 6 |
|---|---|---|---|---|---|---|---|
| | Composition (wt%) | | | | | | |
| Anionic Surfactant | MAP *26 | 20 | | 20 | 20 | 20 | 20 |
| Cationic Polymer | Homopolymer of dimethyldiallylammonium chloride *30 | | 5 | 5 | | | 5 |
| Nonionic Polymer | Polyethylene oxide *36 | | | | 5 | | |
| Anionic Polymer | Sodium arginate *37 | | | | | 5 | |
| oil | Polydimethyl siloxane (90cps) *33 | 5 | 5 | | 5 | 5 | |
| | Carnauba wax (>10000mPa·s) *38 | | | | | | 5 |
| | Purified Water | Balance | | | | | |
| | Evaluation | | | | | | |
| Precipitation characteristics when diluting ** | | N | N | P | N | N | P |
| Skin cleansing ability | | ○ | × | ○ | ○ | ○ | ○ |
| Residual property of the oil | | × | × | × | × | × | × |
| Feel of the skin after washing | | × | Δ | × | × | × | × |
| Moisturizing effect | | × | Δ | × | × | × | × |
| ** P:Present, N:None. | | | | | | | |

Table B3

| Comparative Example No. | 7 | 8 | 9 | 10 |
|---|---|---|---|---|
| Composition (wt%) | | | | |
| MAP *26 | | 6 | 10 | 10 |
| Potassium N-coconut oil fatty acid acyl L-glutamate *28 | | 4 | | |
| Sodium α - olefin sulfonate *39 | 15 | | | |
| Betaine type amphoteric surfactant *40 | 3 | | | |
| Salt of N-Lauryl-β-aminopropionic acid *41 | | 5 | 5 | 5 |
| POE(40) hydrogenated castor oil tristearate | 5 | | | |
| Copolymer of dimethyldiallylammonium chloride (Cationic charge density: 0.0033) *42 | | | 0.2 | |
| Cationic cellulose (Cationic charge density: 0.0026) *31 | | 0.2 | | 0.2 |
| Dimethyl polysiloxane (5cps) | 5 | | | |
| Maleic soybean oil *43 | | 12 | 10 | 11 |
| Myristic acid *44 | | 1 | 2 | 2 |
| Sodium citrate *45 | | 5 | 4 | 3 |
| Purified Water | Balance | | | |
| Evaluation | | | | |
| Precipitation characteristics when diluting ** | N | N | N | N |
| Skin cleansing ability | ○ | Δ | Δ | Δ |
| Residual property of the oil | × | × | × | × |
| Feel of the skin after washing | Δ | Δ | Δ | Δ |

(continued)

| Comparative Example No. | 7 | 8 | 9 | 10 |
|---|---|---|---|---|
| Moisturizing effect | Δ | Δ | Δ | Δ |
| ** P:Present. N:None | | | | |

(Evaluation)

With regard to the compositions obtained in each reference example and comparative example, precipitation characteristics when the composition was diluted, skin cleansing ability, the residual property of the oil, feel of the skin after washing and moisturizing effect were evaluated by the following procedures. The results are shown in Tables B1 to B3.

(1) Precipitation characteristics when diluting

Presence or absence of the precipitate was judged in the same manner as that of Evaluation (1) of the Example A series.

(2) Skin cleansing ability

The cleansing ability was judged in the same manner as that of Evaluation (2) of the Example A series.

(3) Residual property of the oil (marker method)

The residual property of the oil was evaluated in the same manner as that of Evaluation (4) "Residual property of the powder" of the Example A series.

(4) Feel of the skin after washing

The inner side of the human fore-arm was washed by the skin cleansing composition. An expert panelist evaluated the feel of the skin at 5 minutes after towel-drying in contrast with the following criterion by sensory evaluation.

<Criterion for Evaluation>

○: Good. A comfortable touch such as a smooth, or moisturized feeling is recognized.
Δ: Normal. There is a little unnatural feeling, but acceptable. Or it is comparable to feel after washing by a solid soap commercially available.
×: Bad. Uncomfortable feel such as sticky or closed feeling is recognized.

(5) Moisturizing effect

The face was washed by the skin cleansing composition obtained. An expert panelist evaluated a moisturizing effect at 5 minutes after towel-drying in contrast with the following criterion by sensory evaluation.

<Criterion for Evaluation>

○: Enough moisturized feeling.
Δ: Insufficient moisturized feeling.
×: Rough feeling.

17

(Result of the evaluation)

Every skin cleansing composition obtained in the reference examples of the B series was evidenced that it has the skin cleansing ability to wash away soils on the skin such as the sebum, and also has an ability of allowing a prescribed oil to remain on the skin after washing with a result that a moisturizing effect and a good feel are provided.

In the Comparative examples B1 to B6, since an essential component of the present invention was absent, the residual property of the oil was failed and the effect of the present invention was not provided.

The Comparative example B7 corresponding to an example described in the above-mentioned Japanese Patent Application Laid-Open (JP-A) No. 1-151510 does not contain the cationic polymer. Although it had the skin cleansing ability, it had no residual property of the oil, thus being inferior in the moisturizing effect and the touch feeling.

In addition, since the amounts of the cationic polymer are small in the Comparative examples B8 to B10 which correspond to the examples described in the above-mentioned WO 92/17166, the residual property of the oil was poor, and the moisturizing effect and the feel were inferior, and also the skin cleansing ability was insufficient.

## Claims

1. A skin cleansing composition **characterized in that** the skin cleansing composition comprises from 5 to 40% by weight of an anionic surfactant, from 2 to 20% by weight of at least one cationic polymer selected from a homopolymer or a copolymer of dimethyldiallyammonium chloride, a cationic cellulose and a cationic guar gum, and mixtures thereof and from 0.1 to 20% by weight of a pigment powder, wherein the pigment powder is a flaky pigment powder.

2. The skin cleansing composition according to claim 1, wherein the anionic surfactant is at least one compound selected from an alkyl sulfate, an alkyl ether sulfate, a fatty acid soap, a monoalkyl phosphate, an acyl L-glutamate or an acyl isethionate, and mixtures thereof.

3. The skin cleansing composition according to claim 1 or 2, wherein a cationic charge density of the cationic polymer is 0.001 or more.

4. The skin cleansing composition according to any one of claims 1 to 3, wherein the surface of the flaky pigment powder is processed by a hydrophobic treatment.

## Patentansprüche

1. Hautreinigungszusammensetzung, **dadurch gekennzeichnet, dass** die Hautreinigungszusammensetzung 5 bis 40 Gew.% eines anionischen Tensids, 2 bis 20 Gew.% mindestens eines kationischen Polymers, das aus einem Homopolymer oder einem Copolymer von Dimethyldiallylammoniumchlorid, einer kationischen Cellulose und einem kationischen Guar-Gummi und Mischungen daraus ausgewählt ist, und 0,1 bis 20 Gew.% eines Pigmentpulvers umfasst, worin das Pigmentpulver ein flockenförmiges Pigmentpulver ist.

2. Hautreinigungszusammensetzung gemäß Anspruch 1, worin das anionische Tensid mindestens eine Verbindung ist, die aus einem Alkylsulfat, einem Alkylethersulfat, einer Fettsäure-Seife, einem Monoalkylphosphat, einem Acyl-L-glutamat, einem Acylisethionat und Mischungen davon ausgewählt ist.

3. Hautreinigungszusammensetzung gemäß Anspruch 1 oder 2, worin die kationische Ladungsdichte des kationischen Polymers 0,001 oder mehr ist.

4. Hautreinigungszusammensetzung gemäß mindestens einem der Ansprüche 1 bis 3, wobei die Oberfläche des Pigmentpulvers mit einer hydrophoben Behandlung verarbeitet ist.

## Revendications

1. Composition de nettoyage de la peau **caractérisée en ce que** la composition de nettoyage de la peau comprend de 5 à 40% en poids d'un agent tensioactif anionique, de 2 à 20% en poids d'au moins un polymère cationique choisi parmi un homopolymère ou un copolymère de chlorure de diméthyldiallylammonium, une cellulose cationique et une gomme de guar cationique, et des mélanges de ceux-ci et de 0,1 à 20% en poids d'une poudre de pigment, dans laquelle la poudre de pigment est une poudre de pigment lamellaire.

2.  Composition de nettoyage de la peau selon la revendication 1, dans laquelle l'agent tensioactif anionique est au moins un composé choisi parmi un sulfate d'alkyle, un sulfate d'éther d'alkyle, un savon d'acide gras, un phosphate de monoalkyle, un L-glutamate d'acyle ou un iséthionate d'acyle, et des mélanges de ceux-ci.

3.  Composition de nettoyage de la peau selon la revendication 1 ou 2, dans laquelle une densité de charge cationique du polymère cationique est 0,001 ou plus.

4.  Composition de nettoyage de la peau selon l'une quelconque des revendications 1 à 3, dans laquelle la surface de la poudre de pigment lamellaire est traitée par un traitement hydrophobe.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3489686 A
- US 5037818 A
- JP 55038813 A
- JP 7053340 A
- US 6511669 B
- JP 2001131580 A
- US 5977036 A
- WO 9804237 A
- JP 1151510 A
- WO 9417166 A
- WO 9217166 A